# EUROPEAN PATENT APPLICATION

(11) **EP 3 511 711 A1**
(43) Date of publication of application: **17.07.2019**
(21) Application number: 18206433.7
(22) Date of filing: 22.10.2015
(51) Int. Cl.: G01N 33/50, C07K 14/35

(54) **PEPTIDE COMPOSITION AND USES THEREOF**

(30) Priority: 23.10.2014 EP 14190111
(62) Divisional of application: 15788549.2
(71) Applicant: Qiagen Sciences, LLC, Germantown, MD 20874 (US)
(72) Inventor: Boyle, Jeff, Boyds, 20841 MD (US)
(74) Representative: Banse & Steglich Patentanwälte PartmbB

(57) **Abstract**

Subject of the invention is a composition comprising at least one fragment of the peptide ESAT-6 and at least one fragment of the peptide CFP-10. Preferably, the fragments comprise at least two sets of peptides, a first set comprising at least one peptide of from about 7 to 14 amino acid residues in length and a second set comprising at least one peptide of from 16 amino acid residues or greater. The invention also relates to diagnostic methods using the composition.

## Description

### CROSS REFERENCE TO RELATED APPLICATION

This application claims the benefit of European Patent Application No. EP14190111.6, filed October 23, 2014, which application is incorporated by reference herein in its entirety.

### Background of the invention

The invention relates to the field of immunological-based diagnostic assays including an assay to measure cell-mediated immunoresponsiveness.

Immunological-based diagnostic assays are important tools in detecting a variety of disease conditions. The effectiveness of these types of assays lies in part in the specificity of components within the immune system. Notwithstanding this specificity, immunological-based diagnostics are not necessarily always sensitive enough to detect low grade infection or the presence of a persistent low level infection or in subjects with active or latent infectious disease states. There is a need to develop diagnostic assays with enhanced sensitivity in relation to cell-mediated immunoresponsiveness.

One form of immunological-based diagnostic assay involves the stimulation of T-cells within antigens or mitogens in either isolated cell culture or in whole blood culture followed by the detection of effector molecules such as cytokines produced by the stimulated T-cells (also referred to as effector T-cells). The effector molecules are generally detected using techniques such as enzyme immunoassays, multiplex bead analysis, ELISpot and flow cytometry. However, such assays are often not sensitive and selective enough for routine diagnostic application.

The ability to quickly assess cell-mediated immunity and with a high degree of sensitivity is of clinical importance. This is particularly the case with immune system compromised patients. A clinician needs to have an appreciation of the development of a disease state and its effect on the host's immune system.

WO2013/000021 A1 discloses an improved cell mediated immune response assay with enhanced sensitivity. The method for measuring cell-mediated immune response activity in a subject comprises contacting lymphocytes from the subject with at least two sets of peptides, a first set comprising at least one peptide of from about 7 to 14 amino acid residues in length and a second set comprising at least one peptide of from 16 amino acid residues or greater which peptides encompass all or part of a protein antigen. Various protein antigens have been described in the art which induce a cellular immune reaction. For example, WO95/01441 A1 discloses a peptide antigen ESAT-6 isolated from mycobacteria. WO97/09429 A2 discloses a peptide antigen CFP-10 isolated from mycobacteria.

Laurens et al. (Clin. and Diagn. Lab. Imm., March 2000, Vol. 7 No. 2, p155-160) describe a method of diagnosing tuberculosis based on ESAT-6 and CFP-10. They find that a combination of both antigens increase sensitivity and specificity of in vitro and in vivo diagnostic assays when compared to use the isolated antigens.

WO2008/141226 discloses bacterial expression systems for various antigens, such as CFP-10 and ESAT-6. The document does not relate to fragments of the antigens.

WO2009/024822 discloses a list of antigens for use in a vaccine, but does not disclose or suggest specifically combining CFP-10 and ESAT-10 or fragments thereof.

WO2014/085713A1 relates to methods and compositions for detecting tuberculosis based on marker proteins. A diagnostic assay is described in which large fragments of ESAT-6 and CFP-10 are obtained in a probe by trypsin digestion. The document does not disclose defined sets of peptides or methods in which such peptides interact with lymphocyte cells.

However, there is an ongoing need for novel sensitive and selective antigen compositions and corresponding immunological-based diagnostic assays.

### Problem underlying the invention

The problem underlying the invention is to provide novel sensitive and selective antigen compositions and corresponding immunological-based diagnostic assays. Further, the problem is to provide an improved composition and assay for diagnosing tuberculosis.

### Disclosure of the invention

Surprisingly, it was found that the problem underlying the invention is overcome by compositions and methods according to the claims. Further embodiments of the invention are outlined throughout the description.

Subject of the invention is a composition comprising at least one fragment of ESAT-6 and at least one fragment of CFP-10.

As used herein, a "fragment" is a peptide having less amino acids than full length ESAT-6 or CFP-10.

The inventive composition comprises fragments of ESAT-6 and CFP-10. However, it is not excluded that other fragments or antigens are present. In an embodiment of the invention, no other fragments and/or antigens are present. In this embodiment, the immunogenic peptides in the composition are all fragments of ESAT-6 and CFP-10.

In a preferred embodiment, the fragments comprise at least two sets of peptides, a first set comprising at least one peptide of from about 7 to 14 amino acid residues in length and a second set comprising at least one peptide of from 16 amino acid residues or greater.

In this embodiment, the first set of peptides of from about 7 to 14 amino acid residues in length could be regarded as "the short peptides", whereas the second set comprising at least one peptide of from 16 amino acid residues or greater could be regarded as "the long peptides".

In a preferred embodiment, the peptides encompass part of the protein antigen ESAT-6 and CFP-10. In an embodiment, the first and the second set of peptides each comprise fragments of ESAT-6 and CFP-10.

According to the invention, it is not excluded that other fragments of ESAT-6 and CFP-10 are part of the composition. Thus, fragments of ESAT-6 and CFP-10 may also be present having a length of up to 6 amino acids, or 15 amino acids.

In a preferred embodiment, no full length ESAT-6 and/or CFP-10 are part of the composition. In other embodiments, no fragments of ESAT-6 and/or CFP-10 are included, which have a length of more than 75, more than 50 or more than 25 amino acids.

ESAT-6 (UniProt accession no: P0A564; Entrez accession no: 886209) is a 6 kDa early secretary antigenic target of M. tuberculosis. WO95/01441 A1 discloses the protein sequence and a method for isolation from mycobacteria.

CFP-10 (UniProt accession no: P0A566; Entrez accession no: 886194) is a 10 kDa protein also known as ESAT-6-like protein eesxB and secreted antigenic protein MTSA-10. WO97/09429 A2 discloses the sequence and a method for isolating CFP-10 from mycobacteria.

Laurens et al. (Clin. and Diagn. Lab. Imm., March 2000, Vol. 7 No. 2, p155-160) describe a method of diagnosing tuberculosis based on ESAT-6 and CFP-10. They find that a combination of both antigens increase sensitivity and specificity of in vitro and in vivo diagnostic assays when compared to use the isolated antigens. However, the prior art has not described an inventive composition, which combines fragments of ESAT-6 and CFP-10 for inducing cellular immune response.

By "about 7 to 14 amino acids" means 7, 8, 9, 10, 11, 12, 13 or 14 amino acids. This is considered herein a first set of peptides.

By "greater than 15 amino acids" means from 16 to the entire length of the protein antigen, including from 16 to 50 amino acids. This is considered a second set of peptides. The present method is not to be limited to which set of peptides is referred to as first or second. Each set comprises from at least one peptide to a series of overlapping peptides. The lengths of the "long peptides" having 16 amino acids or more are limited by the lengths of ESAT-6 (95 amino acids) and CFP-10 (100 amino acids). In specific embodiments, the maximum lengths of the long peptides are below 90, below 75, below 50 or below 30 amino acids. The peptides can be obtained by known means, for example recombinant peptide production methods in vitro or in vivo, or by digestion of ESAT-6, CFP-10 or derivatives thereof.

The co-incubation of the 7 to 14 amino acid peptides and the greater than 15 amino acid peptides derived from the protein antigen with the lymphocytes results in a more sensitive assay, enabling earlier detection of lymphocyte stimulation than would otherwise be possible. The increased sensitivity includes at least a 10% increased detection of effector molecules compared to co-incubation with a single peptide in the 7 to 14 amino acid range or >15 amino acid range derived from the antigen or the whole antigen itself. The ability to increase the sensitivity of a cell-mediated immune response assay also enables less sensitive means of detection of effector molecules. Furthermore, the magnitude of the cell-mediated immune response detected in the assay presently disclosed can be correlated to the disease state, progression and/or severity. Hence, the present disclosure teaches an assay of a cell-mediate immunoresponsiveness in a subject.

Without limiting the present invention to any one theory or mode of action, the two sets of peptides, the 7 to 14mer peptides and >15mer peptides enables detection by both CD4⁺ and CD8⁺ T-cells. The CD4⁺ T-cells recognize the >15mer peptides and CD8⁺ T-cells recognize the 7 to 14mer peptides. These peptides may be referred to herein as "CD4⁺ peptides" (> 15mer peptides) or "CD8⁺ peptides" (7 to 14mer peptides).

Preferably, the composition comprises multiple peptides of from about 7 to 14 amino acid residues and multiply different peptides of from 16 amino acid residues or greater. In this respect, "multiple peptides" refers to a pool, which may comprise for example from 2 to 2000, preferably from 10 to 1000 or from 20 to 500 different peptides. When multiple peptides are comprised, the immune response is often improved.

In a preferred embodiment, the composition further comprises at least one sugar. Preferably, the sugar is a non-reducing sugar, most preferably trehalose.

It has been described in the art, for example WO2004/042396A1, that sugars may enhance the sensitivity of such methods and compositions.

In a preferred embodiment, the sugar is a non-reducing sugar. A "non-reducing sugar" in particular refers to a sugar which does not react with a detection reagent for reducing sugars, such as Fehling's solution, Benedict's reagent or Tollens' reagent. A non-reducing sugar does not comprise a free reducing end and accordingly, does not comprise a free aldehyde or free ketone group. The non-reducing sugar may have any length and may be linear or branched. In certain embodiments, the non-reducing sugar comprises at least two monosaccharide units. According to one embodiment, in any and all of the monosaccharide units of the non-reducing sugar the carbon atoms neighboring the oxygen atom in the ring structure do not comprise a hydroxyl group and thus, do not comprise an anomeric hydroxyl group. According to one embodiment, the ring structures of the monosaccharide units of the non-reducing oligosaccharide do not comprise a hemiacetal or hemiketal group. According to one embodiment, the non-reducing sugar is an oligosaccharide which comprises 10 monosaccharide units or less, more preferably 8 monosaccharide units or less, 6 monosaccharide units or less, 5 monosaccharide units or less, 4 monosaccharide units or less, 3 monosaccharide units or less or 2 monosaccharide units. Preferably, the non-reducing sugar is a disaccharide. According to one embodiment, the glycosydic bonds are formed between the monosaccharide units by attaching the reducing end of one monosaccharide unit to the reducing end of another monosaccharide unit. Preferred examples of the non-reducing sugar are sucrose and trehalose. Trehalose is particularly preferred because experiments show that trehalose increases the magnitude of response and thus may increase the assay sensitivity and furthermore, a composition comprising trehalose and an antigen shows excellent storage stability. However, the non-reducing sugar can also be a monosaccharide, wherein, e.g., the reducing end is coupled to and thereby blocked by another chemical entity. Accordingly, the non-reducing sugar may be derivatized. Examples of sugar derivatives are aminosugars wherein one or more hydroxyl group is substituted by an amino group or an acetylamino group. In preferred embodiments, the non-reducing sugar is not substituted and in particular is not derivatized. According to one embodiment, the non-reducing sugar is not a polysaccharide. In certain embodiments, the non-reducing sugar is not bound to a protein, peptide or lipid or other macromolecule. According to one embodiment, the non-reducing sugar is not comprised in a cell culture medium or other medium. According to one embodiment, the non-reducing sugar is not comprised in a liquid. The non-reducing sugar is metabolizable by immune cells comprised in the sample. According to one embodiment, the non-reducing sugar is a non-reducing sugar which when present in an appropriate concentration in the incubation composition comprising the sample and the antigen is capable of increasing the release of interferon gamma by re-stimulated T-cells.

The sugar can be part of the composition. Alternatively, the sugar can be added when the composition is contacted with the lymphocytes.

According to one embodiment, the non-reducing sugar is a non-reducing disaccharide, preferably being selected from trehalose and sucrose. The concentration of the non-reducing sugar in the incubation composition is according to one embodiment at least 1.5 mg/ml, preferably at least 2 mg/ml. Suitable ranges for the concentration of the non-reducing sugar in the incubation composition are also described above and it is referred to the above disclosure.

The composition of the invention can be used as a diagnostic or therapeutic composition. Such uses and applications are outlined further below.

The composition and method of the invention are especially applicable for determining a cell-mediated immune response, preferably as a diagnostic composition or in a diagnostic method.

The composition and method of the invention are especially applicable for diagnosing tuberculosis. Assay for diagnosing tuberculosis with ESAT-6, CFP-10 or a combination thereof have been described in the art.

A method for measuring cell-mediated immune response activity in a subject is therefore provided herein, the method comprising contacting lymphocytes from the subject with a composition of claim 1 and measuring the presence or elevation in the level of an immune effector molecule from immune cells wherein the presence or level of the immune effector molecule is indicative of the level of cell-mediated responsiveness of the subject to the antigen.

Preferably, the subject is a human and the sample is undiluted whole blood. Alternatively, the sample is whole blood which comprises from about 10% to 100% by volume of the sample to be assayed or comprises from about 50% to 100% by volume of the sample to be assayed or comprises from about 80% to 100% by volume of the sample to be assayed. The sample volume may be in microliter or milliliter amounts such as from 0.5µ1 to 5ml. Conveniently, the whole blood is collected in a tube comprising heparin and the immune effector molecule is IFN-γ. Generally, the immune effectors are detected with antibodies specific for same such as using EL1SA or an ELISpot.

One or more further additives can be added and thus be included in the composition. E.g. one or more additives can be added that are necessary or advantageous for sample preparation and/or sample preservation such as e.g. a suitable anticoagulant if the sample is a blood sample. Preferably, the anticoagulant is heparin. Additives should not be comprised in a concentration wherein they could interfere with the cell-mediated immune response. According to one embodiment, no simple sugar is added to the incubation composition in addition to the non-reducing sugar. According to one embodiment, no reducing sugar, in particular no reducing monosaccharide is added to the incubation composition in addition to the non-reducing sugar. The subject may have an infection by a pathogenic agent selected from Mycobacterium species such as Mycobacterium tuberculosis or tuberculosis (TB), Staphylococcus species, Streptococcus species, Borrelia species, Escherichia coli, Salmonella species, Clostridium species, Shigella species, Proteus species, Bacillus species, Herpes virus, Hepatitis B or C virus and Human immune deficiency virus (HIV) or a disease resulting therefrom.

A method is also provided of allowing a user to determine the status of cell-mediated immunoresponsiveness of a subject, the method including:
(a) receiving data in the form of levels or concentrations of an immune effector molecule which, relative to a control, provide a correlation as to the state of cell-mediated immunoresponsiveness in a subject, via a communications network, the immune effector molecule measured after exposure of lymphocytes to a composition of the invention;
(b) processing the data via univariate or multivariate analysis to provide an immunoresponsiveness value;
(c) determining the status of the subject in accordance with the results of the immunoresponsiveness value in comparison with predetermined values; and
(d) transferring an indication of the status of the subject to the user via the communications network.

In an embodiment, the composition comprises fat least one additional antigen or fragments thereof. Preferably, the additional antigen is a tuberculosis antigen, preferably TB7.7 or TB37.6.

In an embodiment, the lymphocytes are contacted with a combination of CD4⁺ and CD8⁺ peptides.

The terms "T-cells" and "T-lymphocytes" are used interchangeably herein. An "immune cell" includes a lymphocyte such as a T-cell.

A "combination" also includes multi-part such as a two-part composition where the agents are provided separately and used or dispensed separately or admixed together prior to dispensation. For example, a multi-part assay pack may have a series of overlapping peptides from about 7 to 14 amino acid residues in length and/or greater than 15 amino acid residues in length which encompass all or part of a protein antigen against which a cell-mediated immune response is to be measured. Hence, this aspect of the present disclosure includes agents dried and loose or immobilized to a compartment wall or solid support in an assay pack.

The present disclosure contemplates sets of peptides. The term "set" may be replaced by other terms such as "pool", "group", "series", "collection" and the like without departing from the method instantly disclosed. Each set comprises at least one peptide and includes in an embodiment a series of overlapping peptides. Hence, a first set may contain a series of overlapping peptides of from 7 to 14 amino acid residues in length. These peptides are recognized by CD8⁺ T-cells, (CD8⁺ peptides). A second set may contain a series of overlapping peptides of greater than 15 amino acid residues in length. These peptides are recognized by CD4⁺ T-cells (CD4⁺ peptides) Both sets of peptides encompasses the entire length of or part of a protein antigen. Furthermore, the peptides do not necessarily have to be overlapping or may overlap by a single amino acid or multiple amino acids.

Reference to a series of overlapping peptides from about 7 to 14 amino acid residues in length which encompass all or part of a protein antigen means a peptide of from about 7 amino acid residues in length to a maximum of 14 amino acid residues which in total span from every amino acid residues which in total span amino acid residues to up to 6 amino acid residues of a protein antigen from its N-terminal end to its C-terminal end or part thereof. Hence, if the length of a given peptide is x amino acid residues in length wherein x is from about 7 to 14, then the extent of overlap between two consecutive peptides is from x-1 to x-6. In an embodiment, the overlap of each consecutive peptide is x-1. A series of overlapping peptides of greater than 15 amino acid residues in length also spans all or part of a protein antigen wherein each peptide is at least 16 amino acid residues in length or up to the length of the full protein antigen. In an embodiment, a peptide of greater than 15 amino acid residues in length is from 16 to 50 amino acids such as 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 amino acid residues. As indicated above, there is no necessity for the peptides to overlap provided there is at least one set of one or more 7 to 14 amino acid peptides and another set of at least one >15mer peptides.

The present disclosure includes the case where each peptide in the series is the same length (i.e. x). However, the series of peptides may comprise a mixture of X₁ , x₂, xⱼ. . . xᵢ peptides where each of xᵢ peptides is from about 7 to 14 amino acid residues in length or greater than 15 amino acid residues in length.

Enabled herein is a method for detecting a cell-mediated immune response in a subject, the method comprising incubating lymphocytes from the subject with an inventive composition and then screening for levels of effector molecules produced by activated lymphocytes.

Lymphocytes are activated by co-incubation with at least two sets of peptides, a first set comprising at least one peptide of from about 7 to 14 amino acid residues in length and a second set comprising at least one peptide of from 16 amino acid residues or greater which peptides encompass all or part of a protein antigen.

The present disclosure teaches augmentation of production of effector molecules from lymphocytes exposed to an inventive composition. Such lymphocytes are "activated" or "stimulated" lymphocytes. The augmentation occurs by exposing the cells to an inventive composition. The level of the response is greater than in the presence of whole antigen or a peptide derived from the antigen which is less than 7 amino acids or greater than 14 amino acids. This enables a more sensitive assay in order to assess the cell-mediated immune responsiveness of a subject. The present disclosure, therefore, enables an assay to detect, assess or otherwise monitor a cell-mediated response in a subject by measuring the presence or level of effector molecules from T-cells stimulated by an inventive composition. The assay also enables earlier detection of cell-mediated responsiveness. In an embodiment, the assay taught therein enhances the sensitivity of a cell-mediated assay which may enable less sensitive detection assays to be employed. Furthermore, the extent or magnitude of the cell-mediated immune response is proposed to be reflective or informative of the state, progression and/or severity of a disease condition. For example, the magnitude of the response may determine if a subject has a latent or active or acute infection or disease condition.

Conveniently, the CD4⁺ and/or CD8⁺ peptides are divided into separate pools of peptides.

Without limiting the present invention to any one theory or mode of action, at least two sets of peptides enables both CD4⁺ and CD8⁺ epitopes to be stimulated. The peptides may be referred to herein as " CD4⁺ peptides" (>15mer peptides) or" CD8⁺ peptides" (7 to 14mer peptides).

An additional agent may also be added to the incubation mixture such as to modulate the activity of regulatory T-cells (T-reg cells). The latter encompasses inhibiting the suppressor function of T-reg cells. Agents which modulate T-reg cells encompassed herein include a CD25 ligand; a sense or antisense oligonucleotide to genetic material encoding JAK1 or TYK2; a neutralizing antibody; a CpG containing oligonucleotide; an oligonucleotide acting as a toll-like receptor (TLR) modulating agent; and other TLR modulating agents.

In a particular embodiment, the T-reg cells are immune response suppressor cells the activity of which is inhibited.

A "CpG molecule" means an oligonucleotide comprising a CpG sequence or motif.

The present disclosure provides a means to determine the responsiveness of cell-mediated immunity in a subject and, in turn, teaches the determination of whether a disease condition or an agent induces or is associated with immunosuppression. The method also enables diagnosis of infectious diseases, pathological conditions, determination of the level of immunocompetence and assessing of immune cell responsiveness to endogenous or exogenous agents as well as assessing exposure to protein toxicants. The assay also enables screening of subjects previously exposed to a particular antigen, such as an antigen associated with a disease, infection or contaminant.

Accordingly, an aspect taught herein contemplates a method for measuring cell-mediated immune response activity in a subject, the method comprising contacting lymphocytes from the subject with an inventive composition and measuring the level of an immune effector molecule produced by immune cells wherein the level of the immune effector molecule is indicative of the level of cell-mediated immunoresponsiveness of the subject.

Another aspect contemplated herein is a method for measuring cell-mediated immune response activity in a subject, the method comprising contacting lymphocytes from the subject with an inventive composition and measuring the elevation in the level of an immune effector molecule from immune cells wherein the level of the immune effector molecule is indicative of the level of cell-mediated responsiveness of the subject wherein the level of responsiveness is indicative of the presence or absence or level or stage of a disease or condition selected from the list comprising an infection by a pathogenic agent, an autoimmune disease, a cancer, an inflammatory condition and exposure to a toxic proteinaceous agent.

Yet another aspect enabled herein is a method for measuring cell-mediated immune response activity in a subject, the method comprising contacting lymphocytes from the subject with an inventive composition and measuring the elevation in the level of an immune effector molecule from immune cells wherein the level of the immune effector molecule is indicative of the level of cell-mediated responsiveness and is indicative of the presence or absence or level or stage of a disease or condition selected from the list comprising an infection by a pathogenic agent, an autoimmune disease, a cancer, an inflammatory condition and exposure to a toxic proteinaceous agent.

Still another aspect taught by the present disclosure is an assay to detect the presence, absence, level or stage of a disease or condition in a subject, the method comprising contacting lymphocytes from the subject with an inventive composition and measuring the elevation in the level of an immune effector molecule from immune cells wherein the level of the immune effector molecule is indicative of the disease or condition.

The present disclosure further contemplates a method for determining whether an agent induces immunosuppression in a subject, the method comprising contacting lymphocytes from the subject after exposure to the agent with an inventive composition and measuring the presence and level of an effector molecule from the lymphocytes wherein the level of the effector molecule determines the level of immunosuppression induced by the agent. In accordance with this aspect, the agent may be a medicament or an environmental toxicant.

In an embodiment, the lymphocytes are comprised within a blood sample. In an embodiment, the blood sample is co-stimulated with an inventive composition.

A use is also provided for an inventive composition in the manufacture of a diagnostic assay of cell-mediated immune responsiveness by the method of incubating lymphocytes with a limiting amount of the agonist and detecting the presence or elevation in an effector molecule.

In another embodiment, taught herein is a method for detecting whether a disease condition is inducing immunosuppression in a subject the method comprising contacting lymphocytes from the subject with a disease condition with an inventive composition and measuring the presence or level of an immune effector molecule from the lymphocytes wherein the level of the immune effector molecule is indicative of the extent of immunosuppression induced or associated with the disease condition.

A use is also provided for an inventive composition in the manufacture of a diagnostic assay of cell-mediated immune responsiveness. Generally, the method comprising incubating lymphocytes with an inventive composition.

This use includes the use for detecting or monitoring the presence, absence, level or stage of a disease or condition such as an infection by a pathogenic agent, an autoimmune disease, a cancer, an inflammatory condition and/or exposure to a medicament or a toxic proteinaceous agent such as an environmental toxicant. Measuring "an immune effector molecule" includes measuring one or more different types of molecules.

The present disclosure further enables a method for measuring cell-mediated immune response activity in a subject, the method comprising contacting a regulatory T-cell from the subject with an agent selected from (i) an inhibitor of suppressor regulatory T-cells; and (ii) an activator of immune augmenting cells or a subset thereof; and further contacting T-cells with an inventive composition and measuring the elevation in the level of an immune effector molecule from immune cells wherein the level of the immune effector molecule is indicative of the level of cell-mediated responsiveness of the subject.

Examples of inhibitors or modulators of T-reg function include CD25 ligands such as but not limited to a polyclonal or monoclonal antibody to CD25 or an antigen-binding fragment thereof, humanized or deimmunized polyclonal or monoclonal antibodies to CD25 or a recombinant or synthetic form of the polyclonal or monoclonal antibodies. Other examples of agents include sense or antisense nucleic and molecules directed to the mRNA or DNA (i.e. genetic material) encoding Janus Tyrosine Kinase 1 (JAK1) or Tyrosine Kinase 2 (TYK2) or small molecule inhibitors of JAK1 or TYK2 proteins. Reference to "small molecules" includes immunoglobulin new antigen receptors (IgNARs) as described in International Patent Publication No. WO 2005/1 18629. Yet still further examples of suitable agents stimulating agents such as CpG molecules which act via Tolllike receptors (TLRs) and/or other mechanisms. Hence, CpG containing oligonucleotides and an oligonucleotide acting as a TLR modulating agent also form part of the present disclosure.

A single type of agent may be used or two or more types of agents may be employed to modulate T-reg cells. For example, the assay may be conducted with a CD25 ligand and a JAK1/TYK2 sense or antisense oligonucleotide; a CD25 ligand and a TLR modulating agent; a JAK1/TYK2 sense or antisense oligonucleotide and a TLR modulating agent; or a CD25 ligand, a JAK1/TY 2 sense or antisense oligonucleotide and a TLR modulating agent. Alternatively, just one type of agent is employed. In another alternative, a CpG comprising oligonucleotide and a TLR modulating agent is used.

Reference to a "subject" includes a human or non-human species including primates, livestock animals (e.g. sheep, cows, pigs, horses, donkey, goats), laboratory test animals (e.g. mice, rats, rabbits, guinea pigs, hamsters), companion animals (e.g. dogs, cats), avian species (e.g. poultry birds, aviary birds), reptiles and amphibians. The present subject matter has applicability in human medicine as well as having livestock and veterinary and wild-life applications which includes the horse, dog and camel racing industries. For example, the assay of the present disclosure may be routinely carried out on horses before and/or after heavy exertion (such as a race) to screen for evidence of exercise-induced pulmonary hemorrhage (EIPH). All horses exhibit some form of EIPH to some degree during exercise. However, sub-clinical forms of EIPH can be hard to detect.

Reference to a "human" includes particular populations of humans such as pediatric, elderly and infirmed populations of humans as well as particular cohorts or populations of humans of a particular ethnicity.

In another embodiment, the subject is a human and the cell-mediated immune response assay is used in screening for responsiveness to pathogenic microorganisms, viruses and parasites, potential for development or monitoring autoimmune conditions, Celiac's disease, monitoring a subject's response to oncological challenge and for determining the presence of any immunodeficiency or immunosuppression. The latter may occur, for example, due to certain medicaments including various chemotherapeutic agents. Alternatively, exposure to environmental proteinaceous toxicants and pollutants.

The immune effector molecules may be any of a range of molecules which are produced in response to cell activation or stimulation by an antigen. Although an interferon (IFN) such as IFN-γ is a particularly useful immune effector molecule, others include a range of cytokines such as interleukins (IL), e.g. IL-2, IL-4, IL-6, IL-6 (CXCL8), IL-10, IL-12, IL-13, IL-16 (LCF) or IL-17, IL-la (IL-1F1), IL-I β (IL-1 F2), IL-Ir (IL-1F3), Tumor Necrosis Factor alpha (TNF-a), Transforming Growth Factor beta (TGF-β), a Colony Stimulating Factor (CSF) such as Granulocyte (G)-CSF or Granulocyte Macrophage (GM)-CSF, complement component 5a (C5a), Groa (CXCL1), sICAM-1 (CD54), IP-10 (CXCL10), I-TAC (CXCL1 1), MCP-1 (CCL2), MIF (GIF), MIP-Ia (CCL3), MIP-Iβ (CCL4), RANTES (CCL5) or MIG (CXCL9).

The present disclosure also enables a method for measuring cell-mediated immune response activity in a subject, the method comprising contacting lymphocytes from the subject with an inventive composition and measuring the level of an immune effector molecule from immune cells wherein the level of the immune effector molecule is indicative of the level of cell-mediated responsiveness of the subject.

The assay taught herein enables detection of the presence or absence or level or stage of a disease or condition in a subject such as infection by a pathogenic agent, an autoimmune disease, cancer, exposure to an inflammatory agent exposure to a medicament, exposure to a toxic proteinaceous agent and immunodeficiency or immunosuppression such as induced by a disease condition.

In an embodiment, the sample collected from the subject is generally deposited into a blood collection tube. A blood collection tube includes a blood draw tube or other similar vessel. Conveniently, when the sample is whole blood, the blood collection tube is heparinized. Alternatively, heparin is added to the tube after the blood is collected.

Notwithstanding that whole blood is particularly contemplated and a most convenient sample, the present disclosure extends to other samples containing immune cells such as lymph fluid, cerebral fluid, tissue fluid and respiratory fluid including nasal and pulmonary fluid as well as samples having undergone cell depletion. Reference to "whole blood" includes whole blood which has not been diluted such as with tissue culture, medium, reagents, excipients, etc. In one embodiment, the term "whole blood" includes an assay sample (i.e. reaction mixture) comprising at least 10% by volume whole blood. Additional agents may be added such as culture media, enzymes, excipients antigen and the like without departing from the sample comprising "whole blood".

Blood volumes may be from about 0.5µ1 to 200ml. The present disclosure also enables the use of acoustic microstreaming to improve the mixing of components in the assay. Acoustic microstreaming is disclosed in International Patent Application No. PCT/AU01/00420.

Hence, contemplated herein is a method of mixing one or more lymphocytes and an inventive composition in a vessel, the method comprising providing from about 0.5µl to 150µl of fluid comprising the components in the vessel so as to establish a discontinuity in acoustic impedance and applying an acoustic signal to cause mixing within the fluid. A second acoustic signal may also be applied, the first and second signals having respective frequencies each selected from about 1 Hz to about 20,000 Hz in an alternating manner to effect chaotic mixing within the fluid.

The use of blood collection tubes is compatible with standard automated laboratory systems and these are amenable to analysis in large-scale and random access sampling. Blood collection tubes also minimize handling costs and reduce laboratory exposure to whole blood and plasma and, hence, reduce the risk of laboratory personnel from contracting a pathogenic agent such as HIV or Hepatitis B virus (HBV) or Hepatitis C virus (HCV).

Combining the incubation step with the collection tube is particularly efficacious and enhances the sensitivity of the assay as does the optional feature of incubating the cells in the presence of a simple sugar such as dextrose or glucose.

The cells of the cell-mediated immune system lose the capacity to mount an immune response in whole blood after extended periods following blood draw from the subject, and responses without intervention are often severely reduced or absent 24 hours following blood draw. The reduction of labor and need for specialized plastic ware allows cell-mediated immune stimulation with the peptide antigens to be performed at the point of care locations such as physicians' offices, clinics, outpatient facilities and veterinary clinics or on farms. Once antigen stimulation is complete, the requirement for fresh and active cells no longer exists. IFN-γ and other cytokines or immune effector molecules are stable in plasma and, thus, the sample can be stored, or shipped without special conditions or rapid time requirements.

The incubation step may be from 1 to 50 hours, such as 1 to 40 hours or 8 to 24 hours. A period of 24 hours is particularly convenient.

The ability to measure cell-mediated immunity is important for assessing a subject's ability to respond to an infection by a pathogenic agent such as a microorganism or virus or parasite, to mount an autoimmune response such as in autoimmune diabetes or to protect against cancers or other oncological conditions or to detect an inflammatory condition or to detect exposure or sensitivity of a subject to a toxic agent such as beryllium. The assay described herein also enables detection of disease conditions which lead to immunosuppression or immunosuppression induced by medicaments Consequently, reference to "measuring a cell-mediated immune response in a subject" includes and encompasses immune diagnosis of infectious and autoimmune diseases, a marker for immunocompetence as well as a marker for inflammatory diseases, cancer and toxic agents. Importantly, the combined innate and/or adaptive immune responsiveness is determined. Furthermore, the ability to use small blood volumes enables assays to be readily conducted on, for example, the pediatric, elderly and infirmed populations. The assay herein enables early detection or more sensitive detection of immunoresponsiveness.

In an embodiment, disease conditions leading to immunosuppression include chronic infection and cancer. Medicaments which can lead to immunosuppression include those used to treat rheumatoid arthritis, cancer and inflammatory bowel disease.

Pathogenic or infectious agents include bacteria, parasites and viruses. Examples of bacteria include Gram positive and Gram negative microorganisms such as Mycobacterium species, Staphylococcus species, Streptococcus species, Escherichia coli, Salmonella species, Clostridium species, Shigella species, Proteus species, Bacillus species, Hemophilus species, Borrelia species amongst others. Mycobacterium tuberculosis is a particularly useful target as well as conditions arising from infection by M. tuberculosis such as tuberculosis (TB). Examples of viruses include Hepatitis virus (Hepatitis B virus and Hepatitis C virus), Herpes virus and Human immune deficiency virus (HIV) as well as diseases resulting therefrom. Parasites include Plasmodium species, ringworm, liver parasites and the like. Other pathogenic agents include eukaryotic cells such as yeasts and fungi.

In an embodiment, the additional tuberculosis antigen is TB7.7 or TB37.6. In an embodiment, the subject is infected with HIV.

The present invention is particularly useful for screening for exposure to M. tuberculosis. Hence, the present disclosure teaches a method for measuring cell-mediated immune response activity in a subject, the method comprising contacting lymphocytes from the subject with an inventive composition, wherein the fragments optionally comprise peptides from an additional antigen is selected TB7.7 and TB37.6 from Mycobacterium tuberculosis and measuring the level of an immune effector molecule produced by immune cells wherein the level of the immune effector molecule is indicative of the level of cell-mediated immunoresponsiveness of the subject to M. tuberculosis.

Autoimmune diseases contemplated herein for detection include inter alia alopecia areata, ankylosing spondylitis, antiphospholipid syndrome, autoimmune Addison's disease multiple sclerosis, autoimmune disease of the adrenal gland, autoimmune hemolytic anemia, autoimmune hepatitis, autoimmune oophoritis and orchitis, Behcet's disease, bullous pemphigoid, cardiomyopathy, celiac sprue-dermatitis, chronic fatigue syndrome (CFIDS), chronic inflammatory demyelinating, chronic inflammatory polyneuropathy, Churg-Strauss syndrome, cicatricial pemphigoid, crest syndrome, cold agglutinin disease, Crohn's disease, dermatitis herpetiformis, discoid lupus, essential mixed cryoglobulinemia, fibromyalgia, glomerulonephritis, Grave's disease, Guillain-Barre, Hashimoto's thyroiditis, idiopathic pulmonary fibrosis, idiopathic thrombocytopenia purpura (ITP), IgA nephropathy, insulin dependent diabetes (Type I), lichen planus, lupus, Meniere's disease, mixed connective tissue disease, multiple sclerosis, myasthenia gravis, myocarditis, pemphigus vulgaris, pernicious anemia, polyarteritis nodosa, polychondritis, polyglancular syndromes, polymyalgia rheumatica, polymyositis and dermatomyositis, primary agammaglobulinemia, primary biliary cirrhosis, psoriasis, Raynaud's phenomenon, Reiter's syndrome, rheumatic fever, rheumatoid arrthritis, sarcoidosis, scleroderma, Sjogren's syndrome, stiff-man syndrome, systemic lupus erythematosus, Takayasu arteritis, temporal arteritis/gianT-cell arteritis, ulcerative colitis, uveitis, vasculitis and vitiligo.

It is generally important to assess the potential or actual cell-mediated responsiveness in subjects exposed to these infectious entities. The method of the present disclosure can also be used to detect the presence or absence of these conditions as well as the level or stage of disease process.

Other disease conditions which can lead to immunosuppression include inflammatory disease conditions.

Examples of inflammatory disease conditions contemplated by the present disclosure include but are not limited to those disease and disorders which result in a response of redness, swelling, pain, and a feeling of heat in certain areas that is meant to protect tissues affected by injury or disease. Inflammatory diseases which can be treated using the methods of the present disclosure include, without being limited to, acne, angina, arthritis, aspiration pneumonia, disease, empyema, gastroenteritis, inflammation, intestinal flu, NEC, necrotizing enterocolitis, pelvic inflammatory disease, pharyngitis, PID, pleurisy, raw throat, redness, rubor, sore throat, stomach flu and urinary tract infections, chronic inflammatory demyelinating polyneuropathy, chronic inflammatory demyelinating polyradiculoneuropathy, chronic inflammatory demyelinating polyneuropathy, chronic inflammatory demyelinating polyradiculoneuropathy. In terms of non-human applications,. the present disclosure extends to detecting EIPH in horses and various conditions in animals such as facial tumor disease in the Tasmanian Devil.

Cancer therapy also is somewhat dependent on cell-mediated immunity and the cancer itself or drugs used to treat cancer can lead to immunosuppression. Cancers contemplated herein include: a group of diseases and disorders that are characterized by uncontrolled cellular growth (e.g. formation of tumor) without any differentiation of those cells into specialized and different cells. Such diseases and disorders include ABL1 protooncogene, AIDS related cancers, acoustic neuroma, acute lymphocytic leukaemia, acute myeloid leukaemia, adenocystic carcinoma, adrenocortical cancer, agnogenic myeloid metaplasia, alopecia, alveolar soft-part sarcoma, anal cancer, angiosarcoma, aplastic anaemia, astrocytoma, ataxia-telangiectasia, basal cell carcinoma (skin), bladder cancer, bone cancers, bowel cancer, brain stem glioma, brain and CNS tumors, breast cancer, CNS tumors, carcinoid tumors, cervical cancer, childhood brain tumors, childhood cancer, childhood leukaemia, childhood soft tissue sarcoma, chondrosarcoma, choriocarcinoma, chronic lymphocytic leukaemia, chronic myeloid leukaemia, colorectal cancers, cutaneous T-Cell lymphoma, dermatofibrosarcoma-protuberans, desmoplastic-small-round-cell-tumor, ductal carcinoma, endocrine cancers, endometrial cancer, ependymoma, esophageal cancer, Ewing's sarcoma, extra-hepatic bile duct cancer, eye cancer, eye: melanoma, retinoblastoma, fallopian tube cancer, fanconi anemia, fibrosarcoma, gall bladder cancer, gastric cancer, gastrointestinal cancers, gastrointestinal-carcinoid-tumor, genitourinary cancers, germ cell tumors, gestational-trophoblastic-disease, glioma, gynaecological cancers, hematological malignancies, hairy cell leukaemia, head and neck cancer, hepatocellular cancer, hereditary breast cancer, histiocytosis, Hodgkin's disease, human papillomavirus, hydatidiform mole, hypercalcemia, hypopharynx cancer, intraocular melanoma, islet cell cancer, Kaposi's sarcoma, kidney cancer, Langerhan's-cell-histiocytosis, laryngeal cancer, leiomyosarcoma, leukemia, Li-Fraumeni syndrome, lip cancer, liposarcoma, liver cancer, lung cancer, lymphedema, lymphoma, Hodgkin's lymphoma, non-Hodgkin's lymphoma, male breast cancer, malignant-rhabdoid-tumor-of-kidney, medulloblastoma, melanoma, merkel cell cancer, mesothelioma, metastatic cancer, mouth cancer, multiple endocrine neoplasia, mycosis fungoides, myelodysplastic syndromes, myeloma, myeloproliferative disorders, nasal cancer, nasopharyngeal cancer, nephroblastoma, neuroblastoma, neurofibromatosis, nijmegen breakage syndrome, non-melanoma skin cancer, non-small-cell-lung-cancer-(NSCLC), ocular cancers, oesophageal cancer, oral cavity cancer, oropharynx cancer, osteosarcoma, ostomy ovarian cancer, pancreas cancer, paranasal cancer, parathyroid cancer, parotid gland cancer, penile cancer, peripheral-neuroectodermal-tumors, pituitary cancer, polycythemia vera, prostate cancer, rare-cancers-and-associated-disorders, renal cell carcinoma, retinoblastoma, rhabdomyosarcoma, Rothmund-Thomson syndrome, salivary gland cancer, sarcoma, schwannoma, Sezary syndrome, skin cancer, small cell lung cancer (SCLC), small intestine cancer, soft tissue sarcoma, spinal cord tumors, squamous-cell-carcinoma-(skin), stomach cancer, synovial sarcoma, testicular cancer, thymus cancer, thyroid cancer, transitional-cell-cancer-(bladder), transitional-cell-cancer-(renal-pelvis-/-ureter), trophoblastic cancer, urethral cancer, urinary system cancer, uroplakins, uterine sarcoma, uterus cancer, vaginal cancer, vulva cancer, Waldenstrom' s-macroglobulinemia and Wilms' tumor.

In the above aspects, the antigen may be derived from the pathogenic agent, be associated with the disease condition or cancer or be the toxicant. Alternatively, the infection, disease condition, cancer or toxicant may suppress cell-mediated immunity in which case any antigen to which the subject has been prior exposed could be employed.

The detection of the immune effector molecules may be measured at the protein or nucleic acid levels. Consequently, reference to "presence or level" of the immune effector molecule includes direct and indirect data. For example, high levels of cytokine mRNA are indirect data showing increased levels of the cytokine.

Ligands to the immune effectors are particularly useful in detecting and/or quantitating these molecules. Antibodies to the immune effectors are particularly useful. Techniques for the assays contemplated herein are known in the art and include, for example, radioimmunoassay, sandwich assays, ELISA and ELISpot. Reference to "antibodies" includes parts of antibodies, mammalianized (e.g. humanized) antibodies, deimmunized antibodies, recombinant or synthetic antibodies and hybrid and single chain antibodies. For skin tests, in humans, humanized or deimmunized antibodies are particularly contemplated herein to detect effector molecules.

Both polyclonal and monoclonal antibodies are obtainable by immunization with the immune effector molecules or antigenic fragments thereof and either type is utilizable for immunoassays. Methods of obtaining both types of sera are well known in the art.

Polyclonal sera are less preferred but are relatively easily prepared by injection of a suitable laboratory animal with an effective amount of the immune effector, or antigenic part thereof, collecting serum from the animal and isolating specific sera by any of the known immunoadsorbent techniques. Although antibodies produced by this method are utilizable in virtually any type of immunoassay, they are generally less favored because of the potential heterogeneity of the product.

The use of monoclonal antibodies in an immunoassay is particularly useful because of the ability to produce them in large quantities and the homogeneity of the product. The preparation of hybridoma cell lines for monoclonal antibody production derived by fusing an immortal cell line and lymphocytes sensitized against the immunogenic preparation can be done by techniques which are well known to those who are skilled in the art.

Another aspect enabled herein, therefore, is a method for detecting an immune effector molecule in a sample comprising lymphocytes from a subject, the method comprising contacting the sample or an aliquot of the sample with an antibody specific for the immune effector molecule or an antigenic fragment thereof for a time and under conditions sufficient for an antibody-effector complex to form, and then detecting the complex wherein the immune effector molecule is generated after incubation of the lymphocytes with an inventive composition.

A "sample" includes whole blood or a fraction thereof comprising lymphocytes. This method includes micro-arrays, macro-arrays and nano-arrays on planar or spherical solid supports. A micro- or macro-array is useful. A "sample" also includes a small volume sample of from about 0.5 µl to 1000 µl including 5µl, 10µl, 20µl, 50µl and 100 µl as well as larger volumes such as from 1ml to about 200ml such as 1ml, 2ml, 5ml, 10ml or 20ml.

A wide range of immunoassay techniques are available as can be seen by reference to U.S. Patent Nos. 4,016,043, 4,424,279 and 4,018,653.

The following is a description of one type of assay. An unlabeled antibody is immobilized on a solid substrate and the sample to be tested for the immune effector molecules (e.g. a cytokine) brought into contact with the bound molecule. After a suitable period of incubation, for a period of time sufficient to allow formation of an antibody- immune effector molecule complex, a second antibody specific to the effector molecule, labeled with a reporter molecule capable of producing a detectable signal, is then added and incubated, allowing time sufficient for the formation of another complex of antibody- effector-labeled antibody. Any unreacted material is washed away, and the presence of the effector molecule is determined by observation of a signal produced by the reporter molecule. The results may either be qualitative, by simple observation of the visible signal, or may be quantitated by comparing with a control sample containing known amounts of antigen. This generalized technique is well known to those skilled in the art as would be any of a number of variations.

In these assays, a first antibody having specificity for the instant immune effectors is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, spheres, discs of microplates, or any other surface suitable for conducting an immunoassay: The binding processes are well known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-120 minutes or where more convenient, overnight) and under suitable conditions (e.g. for about 20°C to about 40°C) to allow binding of any subunit present in the antibody. Following the incubation period, the antibody subunit solid phase is washed and dried and incubated with a second antibody specific for a portion of the effector molecule. The second antibody is linked to a reporter molecule which is used to indicate the binding of the second antibody to the effector molecule.

There are many variations to this assay. One particularly useful variation is a simultaneous assay where all or many of the components are admixed substantially simultaneously. Furthermore, binding of an antibody to a cytokine may be determined by binding of a labeled antibody directed to the first mentioned antibody.

By "reporter molecule" as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules. Examples of suitable fluorophores are provided in Table 1. In the I case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labeled antibody is added to the first antibody-antigen complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of antigen which was present in the sample. Again, the present disclosure extends to a substantially simultaneous assay.

Alternately, fluorescent compounds, such as fluorescein and rhodamine, may be chemically coupled to antibodies without altering their binding capacity. When activated by illumination with light of a particular wavelength, the fluorochrome-labeled antibody adsorbs the light energy, inducing a state to excitability in the molecule, followed by emission of the light at a characteristic color visually detectable with a light microscope. The fluorescent labeled antibody is allowed to bind to the first antibody-antigen complex. After washing off the unbound reagent, the remaining tertiary complex is then exposed to the light of the appropriate wavelength the fluorescence observed indicates the presence of the antigen of interest. Immunofluorescene and enzyme immunoassay techniques are both very well established in the art and are particularly preferred for the present method. However, other reporter molecules, such as radioisotope, chemiluminescent or bioluminescent molecules, may also be employed.

There are a range of other detection systems which may be employed including colloidal gold and all such detection systems are encompassed by the present disclosure.

The present disclosure also contemplates genetic assays such as involving PCR analysis to detect RNA expression products of a genetic sequence encoding an immune effector.

In one embodiment, PCR is conducted using pairs of primers, one or both of which are generally labeled with the same or a different reporter molecule capable of giving a distinguishable signal. The use of fluorophores is particularly useful in the practice of the present disclosure. Examples of suitable fluorophores may be selected from the list given in Table 1. Other labels include luminescence and phosphorescence as well as infrared dyes. These dyes or fluorophores may also be used as reporter molecules for antibodies.

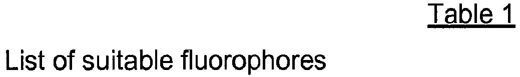

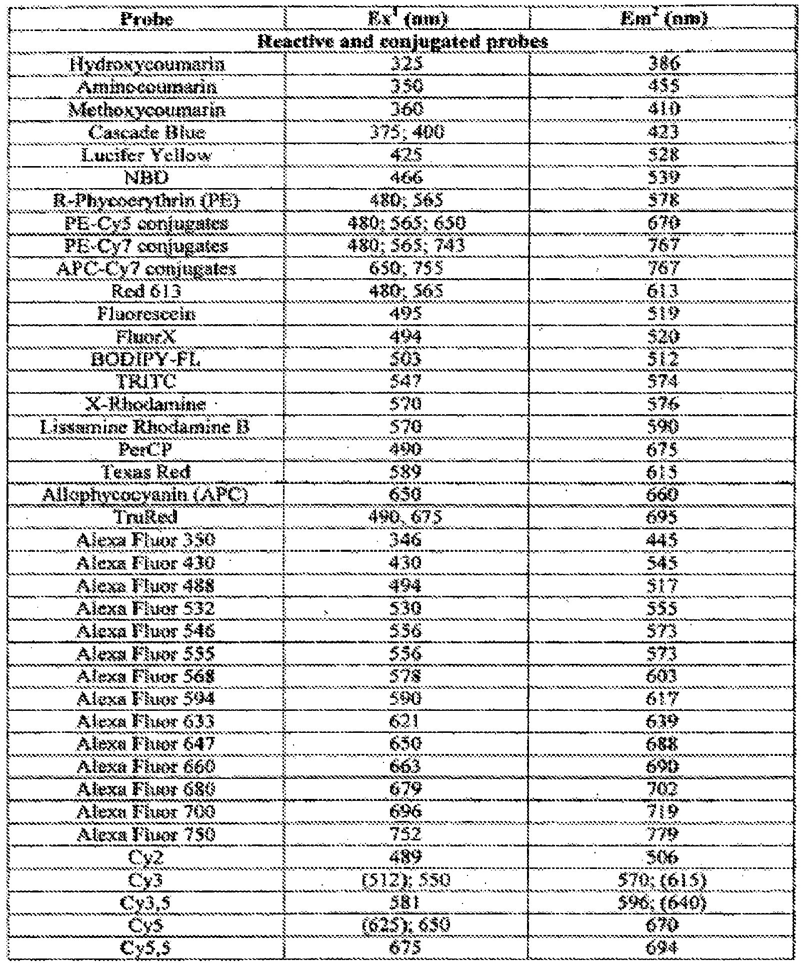

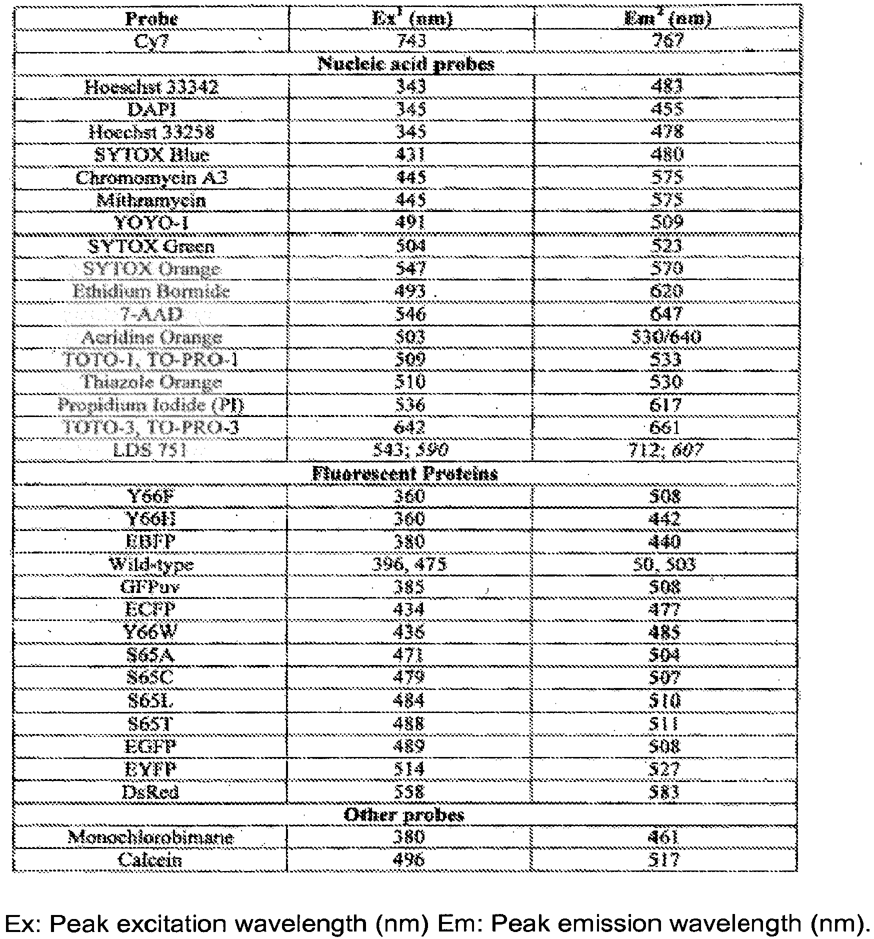

Any suitable method of analyzing fluorescence emission is encompassed herein. In this regard, techniques taught herein include but are not restricted to 2-photon and 3-photon time resolved fluorescence spectroscopy as, for example, disclosed by Lakowicz et al. (1997) Biophys. J. 72:567, fluorescence lifetime imaging as, for example, disclosed by Eriksson et al. (1993) Biophys. J. 2:64 and fluorescence resonance energy transfer as, for example, disclosed by Youvan et al. (1997) Biotechnology et elia 3: 1-18.

Luminescence and phosphorescence may result respectively from a suitable luminescent or phosphorescent label as is known in the art. Any optical means of identifying such label may be used in this regard.

Infrared radiation may result from a suitable infrared dye. Exemplary infrared dyes that may be employed in the present disclosure include but are not limited to those disclosed in Lewis et al. (1999) Dyes Pigm. 42(2): 197, Tawa et al. Mater. Res. Soc. Symp. Proc.488 [Electrical, Optical and Magnetic Properties of Organic Solid-State Materials IV], 885-890, Daneshvar et al. (1999) J. Immunol. Methods 226(1-2): 1 19-128, Rapaport et al. (1999) Appl. Phys. Lett. 74(3) :329-33 \ and Durig et al. (1993) J Raman Spectrosc. 24(5):2 1-285. Any suitable infrared spectroscopic method may be employed to interrogate the infrared dye. For instance, fourier transform infrared spectroscopy as, for example, described by Rahman et al. (1998) J. Org. Chem. 63:6196 may be used in this regard.

Suitably, electromagnetic scattering may result from diffraction, reflection, polarization or refraction of the incident electromagnetic radiation including light and X-rays. Such scattering can be used to quantitate the level of mRNA or level of protein.

Flow cytometry is particularly useful in analyzing fluorophore emission.

As is known in the art, flow cytometry is a high throughput technique which involves rapidly analyzing the physical and chemical characteristics of particles (e.g. labeled mRNA, DNA or proteins) as they pass through the path of one or more laser beams while suspended in a fluid stream. As each particle intercepts the laser beam, the scattered light and fluorescent light emitted by each cell or particle is detected and recorded using any suitable tracking algorithm as, for example, described hereunder.

A modern flow cytometer is able to perform these tasks up to 100,000 cells/particles s^{"1}. Through the use of an optical array of filters and dichroic mirrors, different wavelengths of fluorescent light can be separated and simultaneously detected. In addition, a number of lasers with different excitation wavelengths may be used. Hence, a variety of fluorophores can be used to target and examine, for example, different immune effectors within a sample or immune effectors from multiple subjects.

Suitable flow cytometers which may be used in the methods of the present disclosure include those which measure five to nine optical parameters (see Table 2) using a single excitation laser, commonly an argon ion air-cooled laser operating at 15 mW on its 488 nm spectral line. More advanced flow cytometers are capable of using multiple excitation lasers such as a HeNe laser (633 nm) or a HeCd laser (325 nm) in addition to the argon ion laser (488 or 514 nm).

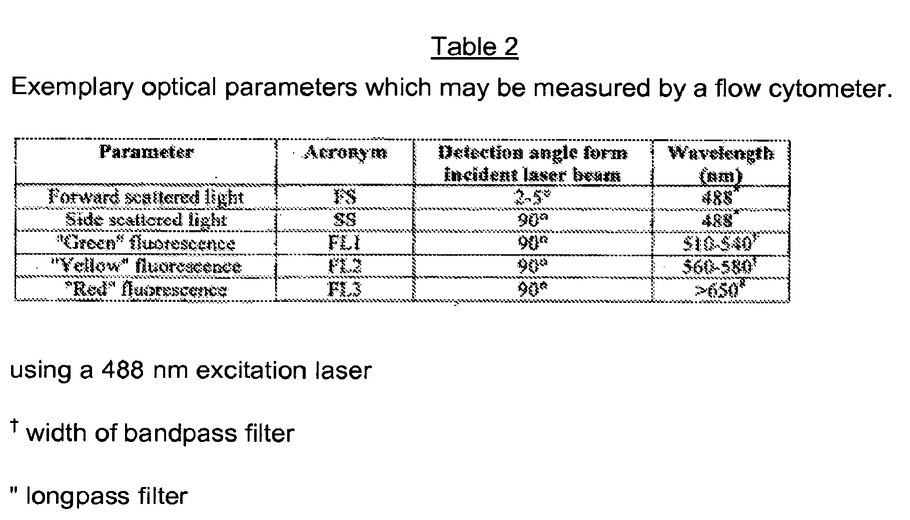

For example, Biggs et al. (1999) Cytometry 36:36-45 have constructed an 1 1 -parameter flow cytometer using three excitation lasers and have demonstrated the use of nine distinguishable fluorophores in addition to forward and side scatter measurements for purposes of immunophenotyping (i.e. classifying) particles. Selection of parameters can be adequately used depends heavily on the extinction coefficients, quantum yields and amount of spectral overlap between all fluorophores (Malemed et al. (1990) "Flow cytometry and sorting", 2nd Ed., New York, Wiley-Liss). It will be understood that the present disclosure is not restricted to any particular flow cytometer or any particular set of parameters. In this regard, the disclosure also contemplates use in place of a conventional flow cytometer, a microfabricated flow cytometer as, for example, disclosed by Fu et al. (1999) Nature Biotechnology 17: 1 109-1 1 1 1.

The assay enabled herein may be automated or semi-automated for high throughput screening or for screening for a number of immune effectors from the one subject. The automation is conveniently controlled by computer software.

The present disclosure further contemplates therefore web-based and non-web-based systems where data on the cell-mediated immunoresponsiveness of a subject are provided by a client server or other architecture platform to a central processor which analyses and compares to a control and optionally considers other information such as patient age, sex, weight and other medical conditions and then provides a report, such as, for example, a risk factor for disease severity or progression or status or an index of probability of disease development. A business method is therefore also provided whereby blood is collected in transportable tubes which is then analyzed for cell-mediated immunoresponsiveness at a defined location and the results then sent in the form of an electronic report via a client server or other architecture platform to a clinical care provider.

Hence, knowledge-based computer software and hardware also form part of the present disclosure. This facilitates clinical care to ascertain whether a disease condition including infection, cancer of inflammation or a medicament or toxicant is inducing or is associated with immunosuppression.

The assays enabled by the instant disclosure may be used in existing or newly developed, knowledge-based architecture or platforms associated with pathology services. For example, results from the assays are transmitted via a communications network (e.g. the internet) or telephone connection to a processing system in which an algorithm is stored and used to generate a predicted posterior probability value which translates to the index of cell-mediated immunoresponsiveness or immunosuppression which is then forwarded to an end user in the form of a diagnostic or predictive report. This report may also form the basis of clinical care management and personalized medicine.

The assay may, therefore, be in the form of a kit or computer-based system which comprises the reagents necessary to detect the concentration of the immune effector molecule following exposure of lymphocytes to an inventive composition and the computer hardware and/or software to facilitate determination and transmission of reports to a clinician.

For example, the present disclosure contemplates a method of allowing a user to determine the status of cell-mediated immunoresponsiveness of a subject, the method including:
(a) receiving data in the form of levels or concentrations of an immune effector molecule which, relative to a control, provide a correlation as the state of cell-mediated immunoresponsiveness in a subject, via a communications network, the immune effector molecule measured after exposure of lymphocytes to an inventive composition;
(b) processing the subject data via univariate or multivariate analysis to provide an immunoresponsiveness value;
(c) determining the status of the subject in accordance with the results of the immunoresponsiveness value in comparison with predetermined values; and
(d) transferring an indication of the status of the subject to the user via the communications network.

Reference to the "univariate" or "multivariate" analysis includes an algorithm which performs the univariate or multivariate analysis function.

Conveniently, the method generally further includes:
(a) having the user determine the data using a remote end station; and
(b) transferring the data from the end station to the base station via the communications network.

The base station can include first and second processing systems, in which case the method can include:
(a) transferring the data to the first processing system;
(b) transferring the data to the second processing system; and
(c) causing the first processing system to perform the univariate or multivariate analysis function to generate the cell-mediated immunoresponsiveness value.

The method may also include:
(a) transferring the results of the univariate or multivariate analysis function to the first processing system; and
(b) causing the first processing system to determine the status of the subject.

In this case, the method also includes at least one of:
(a) transferring the data between the communications network and the first processing system through a first firewall; and
(b) transferring the data between the first and the second processing systems through a second firewall.

The second processing system may be coupled to a database adapted to store predetermined data and/or the univariate or multivariate analysis function, the method including:
(a) querying the database to obtain at least selected predetermined data or access to the univariate or multivariate analysis function from the database; and
(b) comparing the selected predetermined data to the subject data or generating a predicted probability index of a level of cellular immunoresponsiveness or immunosuppression.

The second processing system can be coupled to a database, the method including storing the data in the database.

The method can also include causing the base station to:
(a) determine payment information, the payment information representing the provision of payment by the user; and
(b) perform the comparison in response to the determination of the payment information.

The present disclosure also provides a base station for determining the status of a subject with respect to cell-mediated immunoresponsiveness or immunosuppression, the base station including:
(a) a store method;
(b) a processing system, the processing system being adapted to:
   (c) receive subject data from the user via a communications network, the data including levels of immune effector molecule wherein the level of the effector molecule relative to a control provides a correlation to the state of cell-mediated immunoresponsiveness wherein the immune effector molecule is determined after exposure of lymphocytes to an inventive composition;
   (d) performing an algorithmic function including comparing the data to predetermined data;
   (e) determining the status of the subject in accordance with the results of the algorithmic function including the comparison; and
(c) output an indication of the status of the subject to the user via the communications network.

The processing system can be adapted to receive data from a remote end station adapted to determine the data.

The processing system may include:
(a) a first processing system adapted to:
   (i) receive the data; and
   (ii) determine the status of the subject in accordance with the results of the univariate or multivariate analysis function including comparing the data; and
(b) a second processing system adapted to:
   (i) receive the data from the processing system;
   (ii) perform the univariate or multivariate analysis function including the comparison; and
   (iii) transfer the results to the first processing system.

The processing system can be coupled to a database, the processing system being adapted to store the data in the database.

In accordance with this embodiment, levels of the immune effector molecule may be screened alone or in combination with other biomarkers or disease indicators. An "altered" level means an increase or elevation or a decrease or reduction in the concentrations of the immune effector molecule.

The determination of the concentrations or levels of the immune effector molecule enables establishment of a diagnostic rule based on the concentrations relative to controls. Alternatively, the diagnostic rule is based on the application of a statistical and machine learning algorithm. Such an algorithm uses relationships between effector molecule and disease status observed in training data (with known disease or cell-mediated immunoresponsiveness status) to infer relationships which are then used to predict the status of subjects with unknown status. An algorithm can be employed which provides an index of probability that a subject has a certain level of cell-mediated immunoresponsiveness and/or a disease condition. The algorithm performs a univariate or multivariate analysis function.

Hence, the present disclosure provides a diagnostic rule based on the application of statistical and machine learning algorithms. Such an algorithm uses the relationships between immune effector molecule and level of cell-mediated immunoresponsiveness or immunosuppression observed in training data (with known immune status) to infer relationships which are then used to predict the status of patients with unknown immune status. Practitioners skilled in the art of data analysis recognize that many different forms of inferring relationships in the training data may be used without materially changing the present disclosure.

The present disclosure further contemplates the use of a knowledge base of training data comprising levels of immune effector molecule from a subject with a known cell-mediated immunoresponsiveness level to generate an algorithm which, upon input of a second knowledge base of data comprising levels of the same immune effector molecule from a subject with an unknown immunoresponsiveness level, provides an index of probability that predicts the nature of the cell-mediated immunoresponsiveness.

The term "training data" includes knowledge of levels of immune effector molecule relative to a control wherein the immune effector molecule is determined after exposure of lymphocytes to an inventive composition. A "control" includes a comparison to levels of immune effector molecule in a subject with "normal" immurioresponsiveness or may be a statistically determined level based on trials.

Hence, the term "training data" includes levels of an immune effector molecule.

The levels or concentrations of the immune effector molecule provide the input test data referred to herein as a "second knowledge base of data". The second knowledge base of data either is considered relative to a control or is fed into an algorithm generated by a "first knowledge base of data" which comprise information of the levels of an immune effector in a subject with a known immunological status. The second knowledge base of data is from a subject of unknown status with respect to cell mediated immunoresponsiveness. The output of the algorithm or the comparison to a control is a probability or risk factor, referred to herein as "an index of probability", of a subject having a certain level of immunoresponsiveness or immunosuppressive.

Data generated from the levels of immune effector molecule are input data. The input of data comprising the immune effector levels is compared with a control or is put into the algorithm which provides a risk value of the likelihood that the subject has, for example, an immunosuppressive condition. A treatment regime can also be monitored to ascertain the presence of any immunosuppression. A level of immunosuppression may increase the risk of a subject getting a secondary infection or having a relapse (e.g. during cancer therapy or treatment of a pathogenic infection).

As described above, methods for diagnosing an immunoresponsiveness or immunosuppressive condition by determining the extent to which a subject can mount an innate and/or adaptive immune response via a level of an immune effector molecule provides a second knowledge base data in an algorithm generated with first knowledge base data or levels of the same effector molecule in subjects with a known immune status. Also provided are methods of detecting immunoresponsiveness comprising determining the presence and/or velocity of an immune effector molecule following stimulation of the innate and/or adaptive immune system in a subject's sample. By "velocity" it is meant the change in the concentration of the effector molecule in a subject's sample over time.

As indicated above, the term "sample" as used herein means any sample containing one or more lymphocytes including, but not limited to, whole blood, a whole blood fraction, tissue extracts and freshly harvested cells.

The method of the subject disclosure may be used in the diagnosis and staging of a disease. The present disclosure may also be used to monitor the progression of a condition and to monitor whether a particular treatment is effective or not. In particular, the method can be used to monitor immunosuppression following surgery, cancer therapy or other or medication or exposure to toxicants.

In an embodiment, the subject disclosure contemplates a method for monitoring for immunosuppression in a subject, comprising:
(a) providing a sample from a subject;
(b) determining the level of an immune effector molecule following stimulation by an inventive composition;
   wherein the level of the immune effector relative to a control provides a correlation to the state of cell -mediated immunoresponsiveness and subjecting the levels to an algorithm to provide an index of probability of the subject having a certain level of immunoresponsiveness; and
(c) repeating steps (a) and (b) at a later point in time and comparing the result of step (b) with the result of step (c) wherein a difference in the index of probability is indicative of the progression of the condition in the subject.

Reference to an "algorithm" or "algorithmic functions" as outlined above includes the performance of a univariate or multivariate analysis function. A range of different architectures and platforms may be implemented in addition to those described above. It will be appreciated that any form of architecture suitable for implementing the present disclosure may be used. However, one beneficial technique is the use of distributed architectures. In particular, a number of end stations may be provided at respective geographical locations. This can increase the efficiency of the system by reducing data bandwidth costs and requirements, as well as ensuring that if one base station becomes congested or a fault occurs, other end stations could take over. This also allows load sharing or the like, to ensure access to the system is available at all times.

In this case, it would be necessary to ensure that the base station contains the same information and signature such that different end stations can be used.

It will also be appreciated that in one example, the end stations can be hand-held devices, such as PDAs, mobile phones, or the like, which are capable of transferring the subject data to the base station via a communications network such as the Internet, arid receiving the reports.

In the above aspects, the term "data" means the levels or concentrations of the immune effector following stimulation by a series of overlapping peptides from about 7 to 14 amino acid residues in length which encompass the entire length of a protein antigen. The "communications network" includes the internet and mobile telephone network and telephone land line. When a server is used, it is generally a client server or more particularly a simple object application protocol (SOAP).

One aspect of the present disclosure includes experiments that demonstrate the cell-mediated immune responsiveness of a subject by measuring responsiveness to an inventive composition. In an embodiment, one or more samples such as a sample of peripheral blood, of enriched white
cell fraction of blood or bronchoalveolar lavage may be obtained from a subject having or suspected of development of a particular disease (e.g. autoimmune disease, infection to a pathogenic agent or exposure to a proteinaceous toxicant) and the immune responsiveness measured by determination of effector molecules from effector T-cells (e.g. CD4⁺ T-cells and CD8⁺ T-cells).

The immunobinding methods include methods for detecting or quantifying the amount of a reactive component in a sample, which methods require the detection or quantitation of any immune complexes formed during the binding process. Here, one would obtain a sample suspected of containing a cytokine following stimulation of lymphocytes by an inventive composition and contacting the sample with an antibody and then detecting or quantifying the amount of immune complexes formed under the specific conditions.

Contacting the chosen biological sample with the antibody under conditions effective and for a period of time sufficient to allow the formation of immune complexes (primary immune complexes) is generally a matter of adding the composition to the sample and incubating the mixture for a period of time long enough for the antibodies to form immune complexes with, i.e. to bind to, any effector molecules present. After this time, the sample-antibody composition, such as a tissue section, ELISA plate, ELISpot, dot blot or Western blot, will generally be washed to remove any non-specifically bound antibody species, allowing only those antibodies specifically bound within the primary immune complexes to be detected.

In a particular embodiment, the present disclosure teaches a method for detecting the presence, absence, level or stage of a disease or condition in a human subject, the method comprising contacting whole blood, which comprises at least 10% of the total volume in a reaction mixture, with an inventive composition and measuring the presence or elevation in the level of an immune effector molecule from T-cells wherein the presence or level of the immune effector molecule is indicative of the disease or condition.

In a further embodiment, the present disclosure enables kits for use with the methods described above. In one embodiment, an immunodetection kit is contemplated. In another embodiment, a kit for analysis of a sample from a subject having or suspected of developing a metal or chemically-induced disease is contemplated. In a more particular embodiment, a kit for analysis of a sample from a subject having or suspected of developing a disease is contemplated. In an embodiment, a kit is for assessing the cell-mediated immune responsiveness of a subject before or after a disease state has developed or before or after a subject is given a medicament or is exposed to a toxicant or pollutant. If an antigen is also employed, the kit may also comprise a particular antigen.

The immunodetection reagents of the kit may take any one of a variety of forms, including those detectable labels that are associated with or linked to the given antibody or antigen, and detectable labels that are associated with or attached to a secondary binding ligand. Exemplary secondary ligands are those secondary antibodies that have binding affinity for the first antibody or antigen, and secondary antibodies that have binding affinity for a human antibody.

Further suitable immunodetection reagents for use in the present kits include the two-component reagent that comprises a secondary antibody that has binding affinity for the first antibody or antigen, along with a third antibody that has binding affinity for the second antibody, the third antibody being linked to a detectable label.

The kits may further comprise a suitably aliquoted composition of antigen or effector molecule, whether labeled or unlabeled, as may be used to prepare a standard curve for a detection assay.

The kits may contain antibody-label conjugates either in fully conjugated form, in the form of intermediates, or as separate moieties to be conjugated by the user of the kit. The components of the kits may be packaged either in aqueous media or in lyophilized form.

The container means of any of the kits generally includes at least one vial, test tube, flask, bottle, syringe or other container means, into which the testing agent, the antibody or antigen may be placed, and generally, suitably aliquoted. Where a second or third binding ligand or additional component is provided, the kit will also generally contain a second, third or other additional container into which this ligand or component may be placed. The kits taught by the present disclosure also typically include a means for containing the antibody, peptides derived from an antigen and any other reagent containers in close confinement for commercial sale. Such containers may include injection or blow-molded plastic containers into which the desired vials are retained.

Also contemplated herein is an improved assay to detect a cell-mediated immune response or the level thereof in a subject, the assay comprising incubating lymphocytes from the subject with an antigen and detecting for the presence of or elevation in effector molecules, the improvement comprising incubating the lymphocytes with an inventive composition.

The present disclosure further provides a method of treatment of a subject having a pathogenic infection, an autoimmune disorder or cancer or a propensity for developing such a condition or disorder, the method comprising contacting a source of lymphocytes from the subject with an inventive composition and measuring the presence or elevation in the level of an immune effector molecule from T-cells wherein the presence or level of the immune
effector molecule is indicative of the level of cell-mediated responsiveness of the subject which is indicative of the presence, absence, level or state of the condition or disorder and then treating the condition or disorder.

### BIBLIOGRAPHY

Biggs et al. (1999) Cytometry 36:36-45
Daneshvar et al. (1999) J. Immunol. Methods 226(1-2). 1 19-128
Durig et al. (1993) J. Ram an Spectrosc. 24(5) :2% \-2%5
Eriksson et al. (1993) Biophys. J. 2:64
Fu et al. (1999) Nature Biotechnology 17: 1 109- 1 1 1 1
Lakowicz et al. (1997) Biophys. J. 72:567
Lewis et al. (1999) Dyes Pigm. 42(2): 197
Malemed et al. (1990) "Flow cytometry and sorting", 2nd Ed., New York, Wiley-Liss
Petkovic-Duran et al. (2009) Biotechniques 47:827-834
Rapaport et al. (1999) Appl. Phys: Lett. 7 (3) :329-33 \
Rahman et al. (1998) J. Org. Chem. 63: 196
Tawa et a/. Mater. Res. Soc. Symp. Proc.488 [Electrical, Optical and Magnetic Properties of Organic Solid-State Materials IV], 885-890
Youvan et al. (1997) Biotechnology et elia 3: 1-18

## Claims

1. A composition comprising at least one fragment of ESAT-6 and at least one fragment of CFP-10.

2. The composition according to claim 1, wherein the fragments comprise at least two sets of peptides, a first set comprising at least one peptide of from about 7 to 14 amino acid residues in length and a second set comprising at least one peptide of from 16 amino acid residues or greater.

3. The composition according to at least one of the preceding claims, wherein the 7 to 14 amino acid peptides are recognized by CD8+ lymphocytes and the 16 amino acid or greater peptides are recognized by CD4+ lymphocytes.

4. The composition according to at least one of the preceding claims, further comprising at least one sugar.

5. The composition according to claim 4, wherein the sugar is a non-reducing sugar, preferably trehalose.

6. A process for measuring cell-mediated immune response activity in a subject, preferably a human, said method comprising
contacting lymphocytes from the subject with a composition of at least one of claims 1 to 5, and
measuring the presence or elevation in the level of an immune effector molecule from immune cells,
wherein the presence or level of the immune effector molecule is indicative of the level of cell-mediated responsiveness of the subject.

7. The process according to claim 6, wherein the sample is undiluted whole blood, wherein preferably the whole blood is collected in a tube comprising heparin.

8. The process according to at least one of claims 6 to 7, wherein the immune effector molecule is a cytokine, preferably IFN-γ.

9. The process according to at least one of claims 6 to 8, wherein the immune effectors are detected with antibodies specific for same, preferably using ELISA.

10. The process according to at least one claims 6 to 9, wherein the subject has an infection by a pathogenic agent selected from Mycobacterium species, Staphylococcus species, Streptococcus species, Borrelia species, Escherichia coli, Salmonella species, Clostridium species, Shigella species, Proteus species, Bacillus species, Herpes virus, Hepatitis B or C virus and Human immune deficiency virus (HIV) or a disease resulting therefrom.

11. The process according to at least one of claims 6 to 10, wherein the subject is in a disease condition which is an infection by Mycobacterium tuberculosis or tuberculosis (TB).

12. The process according to at least one of claims 6 to 11, wherein the magnitude of the cell-mediated immune response correlates with the state, progression and/or severity of a disease condition.

13. A method of allowing a user to determine the status of cell-mediated immunoresponsiveness of a subject, the method including:
(a) receiving data in the form of levels or concentrations of an immune effector molecule which relative to a control provide a correlation to the state of cell-mediated immunoresponsiveness from the user via a communications network, the immune effector molecule measured after exposure of lymphocytes to a composition of at least one of claims 1 to 5,
(b) processing the subject data via univariate or multivariate analysis to provide an immunoresponsiveness value;
(c) determining the status of the subject in accordance with the results of the immunoresponsiveness value in comparison with predetermined values; and
(d) transferring an indication of the status of the subject to the user via the communications network.

14. A method according any one of claims 6 to 13, wherein the composition is contacted with the lymphocytes in the presence of at least one sugar, preferably a non-reducing sugar, preferably trehalose.

15. Use of composition of any of claims 1 to 5 or a method of any of claims 6 to 14 for determining a cell-mediated immune response.
